# EUROPEAN PATENT APPLICATION

(11) **EP 1 891 995 A1**
(43) Date of publication of application: **27.02.2008**
(21) Application number: 06118628.4
(22) Date of filing: 08.08.2006
(51) Int. Cl.: A61L 31/14, A61L 31/16, A61K 9/00, A61F 2/00

(54) **Drug loading of porous coating**

(71) Applicant: DEBIOTECH S.A., 1004 Lausanne (CH)
(72) Inventor: Hofmann, Heinrich, 1009, Pully (CH); Piveteau, Laurent-Dominique, 1030, Bussigny (CH); Neftel, Frédéric, 1005, Lausanne (CH)
(74) Representative: Grosfillier, Philippe

(57) **Abstract**

Porous coating for the controlled release of drugs, said coating comprising a porous structure with pores adapted to act as reservoirs or as passages for a drug, in such a way that said drug can move from the external environment to/from or through said pores, characterized by the fact that at least a group of said pores are partially or totally containing an element introduced during or after the loading of the drug in the reservoir pores, in such a way that said drug can still move towards the external environment but in a more restricted manner.

## Description

### Field of invention

The present invention relates to the loading of porous coatings with a drug, the porous coatings having a controlled porosity in pore size and pore distribution. The invention also relates to processes for fabricating such coatings and to objects obtained according to such processes.

### State of the art

A porous coating as defined above is disclosed in patent applications EP 05 108 573.6 filed on September 16, 2005 and EP 06 114 127.1 filed on May 17, 2006 by the same applicant.
This prior art shows a porous coating which may be advantageously used for the slow release of drugs. To this effect, the coating comprises an internal micro-porous structure and an external nano-sized structure, the pore diameter of the internal structure being greater than the pore diameter of the external structure, both porosities interconnecting each other in such a way that a fluid containing a drug can move from the external environment to the micro-pores of the internal structure and vice-versa, the micro-pores of the internal structure acting as drug reservoir.
The presence of two distinct pore sizes is essential to the effectiveness of the coating. In order to store and release over a few days to a few months a drug, the coating must combine two pore sizes: one of large size acting as a reservoir and where the drug is stored and another of size in relation to the released molecules that controls the release of the drug. Micrometer size cavities are created by depositing a template onto the implant. This template is made, for example, of mono disperse particles that are deposited onto the substrate. Nano-openings are created following different approaches. In a first possible embodiment, a second layer of template material is deposited and the nano-openings correspond to the interaction surface between the particles of the two layers. In a second possible embodiment, nano-openings are created by adding a second layer of nano-porous material. Finally the template materials are removed by, for example, a thermal treatment and cavities are created.
Other prior art shows alternative ways of creating a porous coating for drug release applications. For example Reed, Looi and Lye (CA 2 503 625) use a differential attack of a metallic alloy. By removing one component of the alloy, they create a porous layer. Brandau and Fischer (US 6,709,379) create the porosity by an electrolytic oxidation combined with an anodization. Herlein, Kovacs and Wolf (EP 1 319 416) create pores at the surface of a metallic stent through electrochemically induced pitting. However, in all these cases, the created porosity has the disadvantage of being homogeneous in size, or at least having a homogeneous size distribution. As a result, the loaded amount of drug will be either low (small pores) or the release will occur over a short period of time (large pores) i.e. over a few hours only.

Before use, the coating has to be loaded with a drug. Practically, for filling the pores of the internal layer, the drug has to cross the external layer. Loading of drugs into a porous layer has already been described in the literature (see for example R.S. Byrne and P.B. Deasy, International Journal of Pharmaceutics 246 (2002) 61-73). The porous material is soaked into a solution, or covered by a solution of the drug to be loaded. It is maintained into this solution for some time, and after removal the solvent is evaporated. In order to increase the penetration of the solution into the pores, a vacuum is created before the introduction of the material into the solution, is then broken while the material is in the solution, and finally an overpressure is created during the removal of the material out of the solution in order to push the liquid into the pores.
Whichever the size of the external pores is, a problem occurs:
- If the size is very small, drug loading takes a long time (same order of magnitude than the release period) and the repetition of the loading procedure as described above may be difficult.
- If the size is relatively important, drug loading is faster and easier to achieve but the drug is rapidly released.
There is therefore a need to solve the above cited problem.

A possible approach for solving this problem is to load the micro-pores before the nano-pores are created. In a first step, a micro-porosity is created in the coating that will act as a reservoir with its upper part (towards the outside) open with large pores. The drug is then loaded into the micro-pores and a second structure with nano-pores is deposited. However, this approach offers several disadvantages that will differ depending on the material used (ceramic, polymer...) as well as the exact process. In most of the cases, creating the nano-porosity after the filling of the reservoir will create an additional interface between the micro-porous and the nano-porous structures. It is well know from the literature on coatings and thin films that interfaces introduce mechanical weaknesses that may have a dramatic effect on the stability of the coating. If treatments are applied post deposition to overcome this weakness and improve the mechanical binding, being for example thermal or chemical treatments, their effect may have a negative impact on the effectiveness or the safety of the drug that is loaded.

### General Description of the invention

### Short description of the invention

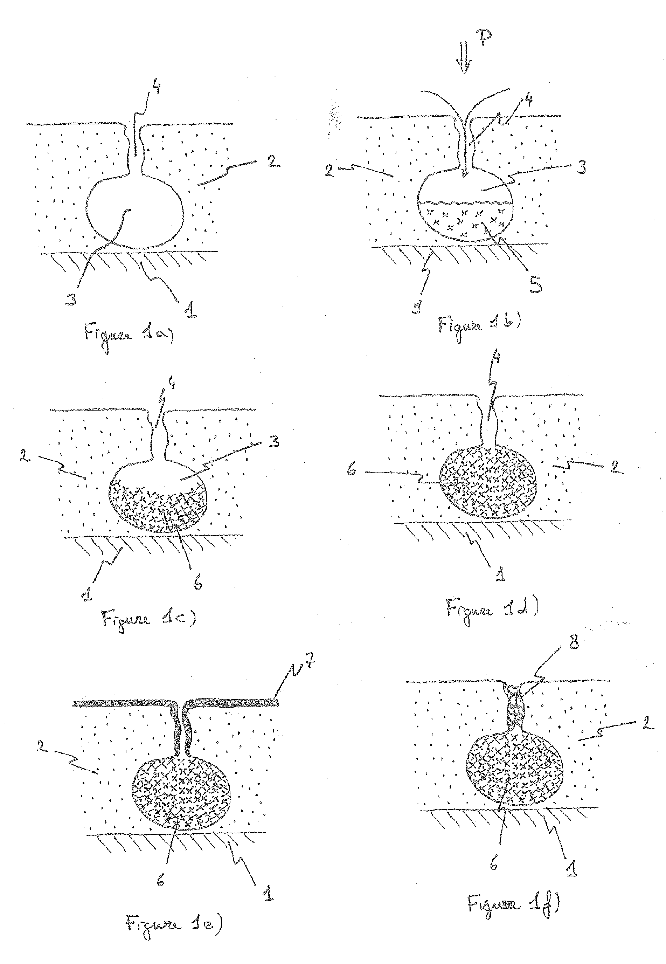

Obtaining simultaneously a faster loading and a slower drug release is achieved with the coating of the present invention which relates to a porous coating for the controlled release of drugs, said coating comprising a porous structure with ports adapted to act as reservoirs or as passages for a drug, in such a way that said drug can move from the external environment to/from or through said pores, characterized by the fact that at least a group of said pores are partially or totally containing an element introduced during or after the loading of the drug in the reservoir pores, in such a way that said drug can still move towards the external environment but in a more restricted manner.
As a non-limiting example, a nano-porous layer is created according to one of the approaches described in EP 05 108 573.6 or EP 06 114 127.1 (figure 1a)). Nano-porosity is chosen to be larger than the size of the molecules to be loaded. The drug is then incorporated using, for example, a dipping of the coated object into a solution, the application of an external pressure (figure 1 b)) and the evaporation of the solvent (figure 1c)). This procedure can be repeated several times if needed (figure 1d)). During of after the loading process, the porosity of the nano-porous layer is reduced and brought back to a size similar to that of the loaded molecule. This reduction in size can be, for example but not exclusively, conducted by a narrowing approach (figure 1e)) or by a plug approach (figure 1f)).

### The narrowing approach (figure 1e))

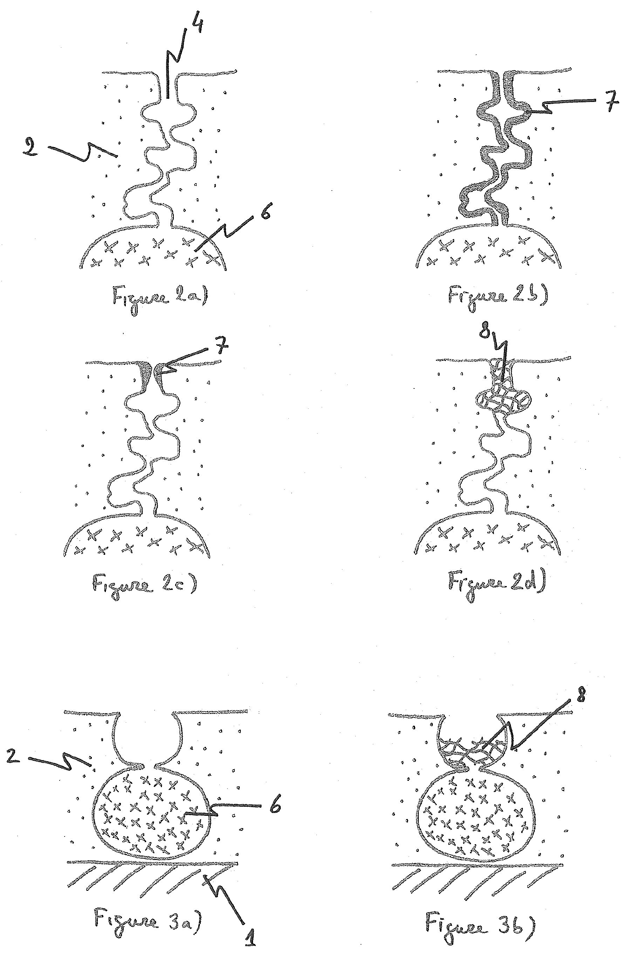

In one possible embodiment, the element is a coating deposited on the pore walls. In a possible embodiment, the nano-porosity is a group of cavities of a few tens of nanometers interconnected by narrow passages a few nanometers (figure 2a)). In the narrowing approach, passages with diameters much larger than the size of the molecule are created. The drug is then loaded into the buried micro-cavities and the passages diameter is reduced by, for example, surface precipitation of an oxide (figure 2b)).
In another possible embodiment, the element can be deposited only on a part of said pore walls (figure 2c)). In a preferred embodiment the element will be deposited on the part of the walls towards the outside of the coating.

The pores diameter of the external layer, without said element, is preferably at least three times greater than the pores diameter with said element.
In a specific embodiment the pores diameter of the external layer, without said element, is about 100 nanometers. In this case, the pores diameter of the external layer, with said element, may be less than 30 nanometers.

### The plug approach (figure 1f))

In another embodiment, the element is a plug having a porosity which smaller than the porosity of the external layer and that is placed next to the outside part of the coating.
In a possible embodiment, the nano-openings are created by a nano-porous layer deposited on top of the internal structure. The nano-porosity is a combination of cavities of a few tens of nanometers interconnected with passages of a few nanometers (figure2a)). The plugs are created, for example, by introducing an adapted sol precursor into the cavities and inducing its in situ gelification (figure 2d)). Drying of this gel can then be conducted under supercritical conditions in order to completely maintain its structures.
In another possible embodiment, the nano-openings are created by the contact surface existing between two particles of the template material (figure 3a)). In this embodiment, the template is made of more than one layer of particles and the coating material is deposited in order to cover all the template particle layers except the upper one that is only partly covered. The plugs are created, for example, by precipitating a nano-powder in the upper cavities. In another possible embodiment, the plugs are created by introducing a sol into the upper cavity and induction gelification (figure 3b)). Drying of this gel can then be conducted under supercritical conditions in order to completely maintain its tri-dimensional structure.

### Process to produce a coating (figures 1a) to 1f))

The invention also encompasses a process for manufacturing a porous coating (2) as previously defined, the process comprising the following steps:
- Providing an internal layer having a first porosity (3) on a substrate (1),
- Providing an external layer having a second porosity (4) on said internal layer, the pores diameter of the external layer being smaller than the pores diameter of the internal layer,
- Filling with a drug the pores of the internal layer, said filling being made through the pores of the external layer,
- Restricting the average cross section of the pores of the external layer, in such a way that said drug can still pass through the pores of the external layer but in a more restricted manner.

In a possible embodiment the filling procedure is done by preparing a solution of the drug to be loaded (5) into the coating, dipping the coating into this solution for a given amount of time, removing the coating from the solution and finally evaporating (6) the remaining solvent. This procedure can be repeated several times.
In another embodiment the drug solution is deposited directly onto the surface of the coating and the solvent is evaporated.

In another embodiment, an electric filed is applied between the coating and the solution. If the drug molecule is electrically loaded, this field will facilitate its integration into the coating.
In a preferred embodiment the filling step includes the use of a pressure gradient between the external environment and the space inside the pores of the internal layer.
In a preferred embodiment the pressure gradient is created by:
- first creating a vacuum around the coating to be loaded
- then dipping the coating into the solution of drug to be loaded
- then breaking the vacuum and creating an overpressure
- finally removing the coating

In one possible embodiment the restriction step is a coating of the pores wall (7).
In a preferred embodiment the coating of the pore walls is done by the surface precipitation of an oxide.
In another preferred embodiment the coating of the pore walls is done by covering the surface of the coating by a sol precursor and inducing local in situ gelification. In a preferred embodiment, this sol precursor is deposited by dip-coating.
In another embodiment the coating of the pore walls is done by vapor deposition, such as for example chemical vapor deposition or physical vapor deposition.
In another embodiment the coating of the pore walls is done by sputtering of a material onto the porous coating.
In a possible embodiment, only the upper part, towards the outside, of the nano-porous coating is restricted in size.

In another possible embodiment the restriction step includes the placement of a plug (8) having a nanoporosity which is smaller than the porosity of the external layer.

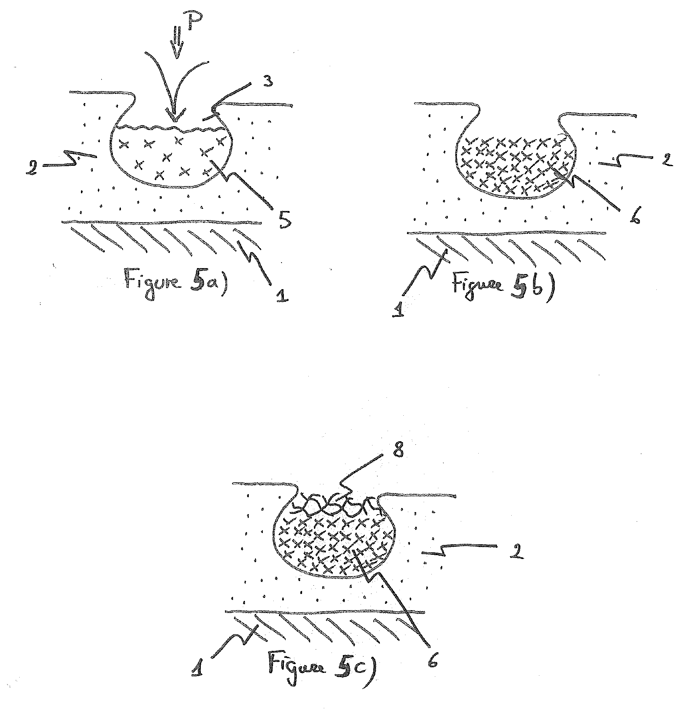

In another possible embodiment, the nano-porous external structure corresponds to the upper part of the internal structure (figure 5a)). After loading of the drug, the upper part of the internal structure, i.e. the external structure, is closed with a plug. This plug can be obtained, for example, by inserting a sol precursor into the external structure and inducing gelification. Drying can be conducted under supercritical conditions.

### Sandwich layer

In another embodiment the internal layer is positively charged , alternately negatively charged, the external layer is neutral and wherein the drug is negatively charged, alternately positively charged.

For the filling, an electric field is applied between the coating and the drug solution. The drug molecules being of the opposite charge than the internal structure, their migration towards the reservoir will be facilitated. The outer layer is maintained neutral, for example by adjusting the pH and bringing it to its isoelectric point. In this way, drug molecules are not disturbed during their migration through the external structure. This would be the case if this external structure was charged. If the molecule is negatively charged, and therefore the internal structure is positively charged, and the external structure is positively charged, molecules will be attracted onto the coating but will have difficulties, due to attraction forces, to go into the reservoir. They will be stuck into the external structure. If now the external structure is negatively charged, the molecules will first feel a repulsion force and not even penetrate into the coating.

### Application

A major application for these objects, as can be readily understood from the different embodiments and variants described above, is in the field of drug eluting medical implants. Of particular interest are stents, orthopedic and dental implants. The porosity is used as a drug reservoir that will release its content in a controlled way over time.

For stents the coating can be loaded with one or several drugs. It can be a combination of the following drugs given as non-exclusive examples: antioxidants, anti-inflammatory agents, anti-coagulant agents, drugs to alter lipid metabolism, anti-proliferatives, anti-neoplastics, tissue growth stimulants, functional protein/factor delivery agents, chemotherapeutic agents, tissue absorption enhancers, anti-adhesion agents, germicides, antiseptics, proteoglycans, GAG's, gene delivery, antifibrotics, anti-migratory agents, pro- healing agents, ECM/protein production inhibitors, cytostatic, proliferation inhibitors, growth inhibitors
More specifically, it can be loaded with specific drugs such as, but non exclusively: Paclitaxel (Taxol), Sirolimus (Rapamycin), Everolimus, Zotarolimus (ABT-578), Tacrolimus, Dexamethasone, Biolimus A9, Des-asparate angiotensin I (DM-1), Sialokinin, Cerivastatin, Cilostazol, Fluvastatin, Lovastatin, Pravastatin, Simvastatin, platin, Oxalyplatin, Platin analogs.

The object can also be an orthopedic or dental implant wherein the pores may be adapted in the same manner as for the stent discussed above. In such case, the porosity obtained can be of interest, for example but not exclusively, to store growth factors such as bone growth factors, increase biocompatibility, avoid infection by storing antibacterial agents, or create regions where bone or cartilaginous tissue can grow and attach in a solid manner to the implant.

Accordingly the support can be made of metal, of ceramic or polymer. It can also be made of biodegradable material.

### Figures

The invention is discussed below in a more detailed way with examples illustrated by the following figures :
Figure 1a schematically shows a possible embodiment of a pore of an internal layer in connection with a pore of the external layer.
Figure 1b shows the filling of the internal pore with a drug in a fluidic state.
Figure 1c represents the solvent evaporation.
Figure 1d shows an internal pore completely filled with a drug after solvent evaporation.
Figure 1e shows a first embodiment of the invention consisting of a coating of the external pore.
Figure 1f shows a second embodiment of the invention consisting of a plug made of nanoporous material.
Figure 2a shows another possible embodiment of a pore of an internal layer in connection with a pore of the external layer.
Figure 2b shows a possible embodiment of the invention consisting of a coating of the external pore.
Figure 2c shows a possible embodiment of the invention consisting of a coating of the external part of the external pore.
Figure 2d shows a possible embodiment of the invention consisting of a plug made of nano-porous material.
Figure 3a shows another possible embodiment of a pore of an internal layer in connection with a pore of the external layer.
Figure 3b shows a possible embodiment of the invention consisting of a plug made of nano-porous material.

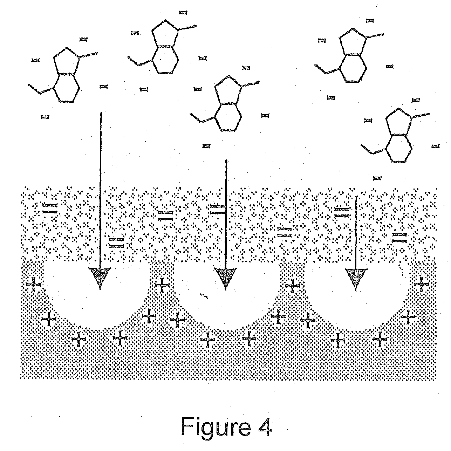

Figure 4 shows a possible embodiment of the invention wherein the internal layer is positively charged, the external is neutral and the molecules are negatively charged.
Figure 5a shows another possible embodiment of a coating wherein the external nano-porous structure is part of the internal structure as well as the filling of the pore with a drug in a fluidic state.
Figure 5b shows a pore completely filled with a drug after solvent evaporation.
Figure 5c shows the pore filled with a drug and covered with a plug made of nanoporous material.

## Claims

1. Porous coating for the controlled release of drugs, said coating comprising a porous structure with pores adapted to act as reservoirs or as passages for a drug, in such a way that said drug can move from the external environment to/from or through said pores, **characterized by** the fact that at least a group of said pores are partially or totally containing an element introduced during or after the loading of the drug in the reservoir pores, in such a way that said drug can still move towards the external environment but in a more restricted manner.

2. Porous coating according to **claim 1** comprising an internal micro-porous structure and an external nano-porous structure, the pore diameter of the internal structure being greater than the pore diameter of the external structure, both porosities interconnecting each other in such a way that a fluid containing a drug can move from the external environment to the pores of the internal structure and vice-versa, **characterized by** the fact that the pores of the internal structure contain a drug and wherein at least the pores of the external structure are partially containing an element introduced during or after the loading of the drug, in such a way that said drug can still pass through said pores but in a more restricted manner.

3. Porous coating according to **claim 2** wherein said external nano-porous structure is a nano-porous layer.

4. Porous coating according to **claim 2** wherein said external nano-porous structure is made of micrometer size pores interconnected to the internal structure by nano-meter size openings.

5. Porous coating according to **anyone of the previous claims** wherein said coating is made of a ceramic such as an oxide, a phosphate, a carbonate, a nitride or a carbonitride.

6. Porous coating according to **anyone of the previous claims 1 to 4** wherein said coating is made of a metal.

7. Porous coating according to **anyone of the previous claims 1 to 4** wherein said coating is made of a polymer or a hydrogel.

8. Porous coating according to **anyone of the previous claims 1 to 4** wherein said coating is made of a biodegradable material.

9. Porous coating according to **anyone of the previous claims** wherein said element is a layer deposited on the pore walls.

10. Porous coating according to **claim 9** wherein said layer is deposited only on a part of said pore walls.

11. Porous coating according to **claims 9 or 10** wherein said layer is deposited by surface precipitation, by a sol-gel route, by CVD, by PDV, by epitaxial growth, by sputtering by ion implantation, by silane grafting or by electrodeposition.

12. Porous coating according to **claim 9 or 10** wherein the minimal pores diameter of the external layer, without said element, is at least two times greater than the minimal pores diameter with said element.

13. Porous coating according to **claim 12** wherein the minimal pores diameter of the external layer, without said element, is about 100 nanometers.

14. Porous coating according to **claim 13** wherein the pores diameter of the external layer, with said element, is less than 50 nanometers.

15. Porous coating according to **anyone of the previous claims** wherein said element is a plug having a mean pore size which is smaller than the mean pore size of the external layer.

16. Porous coating according to **claims 15** wherein said plug is formed by surface precipitation, by a sol-gel route, by CVD, by PDV, by epitaxial growth, by sputtering, by ion implantation, by silane grafting or by electrodeposition.

17. Porous coating according to **anyone of the previous claims** wherein the internal structure is positively charged, alternately negatively charged, and wherein the drug is negatively charged, alternately positively charged.

18. Process for manufacturing a porous coating according to **anyone of the previous claims 1 and 5 to 17** comprising the following steps :
- Providing an layer having a porosity on a substrate,
- Filling with a drug the pores of the layer
- Restricting the average cross section of the pores, in such a way that said drug can still pass through the pores but in a more restricted manner.

19. Process for manufacturing a porous coating according to **anyone of the previous claims 2 to 17** comprising the following steps :
- Providing an internal layer having a first porosity on a substrate,
- Providing an external layer having a second porosity on said internal layer, the pores diameter of the external layer being smaller than the pores diameter of the internal layer,
- Filling with a drug the pores of the internal layer, said filling being made through the pores of the external layer,
- Restricting the average cross section of the pores of the external layer, in such a way that said drug can still pass through the pores of the external layer but in a more restricted manner.

20. Process according to the **claims 18 or 19** wherein the coating is filled with the drug by dipping said coating into a solution containing the drug

21. Process according to the **previous claim** wherein a vacuum is created around the coating and the solution before the dipping of the coating into the solution

22. Process according to any of the **claims 20 or 21** wherein a pressure is applied around the coating and the solution before the pulling of the coating out of the solution.

23. Process according to **claim 18 or 19** wherein an electrical field is created between the internal layer of the coating and the solution to attract the drug molecules and fill the coating.

24. Process according to **claim 18 or 19** wherein the restriction step is a coating of the pores wall.

25. Process according to **claim 18 or 19** wherein the restriction step includes the placement of a plug having a nanoporosity which is smaller than the porosity of the external layer.

26. Process according to **claims 24 or 25** wherein the restriction step is done by surface precipitation, by a sol-gel route, by CVD, by PDV, by epitaxial growth, by sputtering or by electrodeposition.

27. Process for manufacturing a porous coating comprising the following steps :
- Providing an internal layer having a first porosity on a substrate,
- Providing an external layer having a second porosity on said internal layer, the pores diameter of the external layer being smaller than the pores diameter of the internal layer,
- Filling with a drug the pores of the internal layer, said filling being made through the pores of the external layer, **characterized by** the fact that the internal layer is positively charged , alternately negatively charged, the external layer is neutral and wherein the drug is negatively charged, alternately positively charged.

28. Porous coating wherein the internal structure is positively charged, alternately negatively charged, the external structure in neutral, and wherein the drug is negatively charged, alternately positively charged.
